# EUROPEAN PATENT SPECIFICATION

(11) **EP 2 271 778 B1**
(45) Date of publication and mention of the grant of the patent: **16.11.2011**
(21) Application number: 09727056.5
(22) Date of filing: 27.03.2009
(51) Int. Cl.: C12Q 1/70, C12Q 1/68

(54) **METHOD FOR DETECTION OF HERPESVIRUS IN A TEST SAMPLE**
VERFAHREN ZUM NACHWEIS DES HERPESVIRUS IN EINER TESTPROBE
PROCÉDÉ DE DÉTECTION DU VIRUS DE L'HERPÈS DANS UN ÉCHANTILLON A TESTER

(30) Priority: 03.04.2008 GB 0806041
(43) Date of publication of application: 12.01.2011
(73) Proprietor: Genomica S.A.U., 28820 Coslada (ES)
(72) Inventor: MANJÓN, Núria, E-28820 Coslada (ES); VILLAHERMOSA, Maria, L., E-28820 Coslada (ES)
(74) Representative: Fleck, Barbara
(86) International application number: PCT/GB2009/050301
(87) International publication number: WO 2009/122201

(56) References cited:
- WO-A-2007/056463
- WO-A1-89/09282
- WO-A1-94/02634
- WO-A2-03/033735
- WO-A2-2007/113388
- US-A1- 2004 110 195
- US-A1- 2007 207 453
- DATABASE Geneseq [Online] 23 April 1997 (1997-04-23), "Varicella-zoster virus genomic amplification primer." XP002531456 retrieved from EBI accession no. GSN:AAT37529 Database accession no. AAT37529 & WO 96 25909 A2 (INST DE SALUD CARLOS III [ES]; CASAS INMACULADA [ES]; TENORIO ANTONIO) 29 August 1996
- DATABASE Geneseq [Online] 23 April 1997 (1997-04-23), "Varicella-zoster virus genomic amplification primer." XP002531457 retrieved from EBI accession no. GSN:AAT37524 Database accession no. AAT37524
- DATABASE Geneseq [Online] 15 November 2007 (2007-11-15), "Human herpesvirus 2-specific probe SEQ ID No 5." XP002531458 retrieved from EBI accession no. GSN:AJG49149 Database accession no. AJG49149
- DATABASE Geneseq [Online] 23 April 1997 (1997-04-23), "Herpes simplex virus genomic amplification primer." XP002531459 retrieved from EBI accession no. GSN:AAT37528 Database accession no. AAT37528
- MARKOULATOS PANAYOTIS ET AL: "Laboratory diagnosis of common herpesvirus infections of the central nervous system by a multiplex PCR assay" JOURNAL OF CLINICAL MICROBIOLOGY, vol. 39, no. 12, December 2001 (2001-12), pages 4426-4432, XP002531453 ISSN: 0095-1137
- MACKAY IAN M ET AL: "Detection and discrimination of herpes simplex viruses, Haemophilus ducreyi, Treponema pallidum, and Calymmatobacterium (Klebsiella) granulomatis from genital ulcers" CLINICAL INFECTIOUS DISEASES, vol. 42, no. 10, May 2006 (2006-05), pages 1431-1438, XP002531455 ISSN: 1058-4838
- ERHARDT ANDREAS ET AL: "Quantitative assay of PCR-amplified hepatitis B virus DNA using a peroxidase-labelled DNA probe and enhanced chemiluminescence" JOURNAL OF CLINICAL MICROBIOLOGY, vol. 34, no. 8, 1996, pages 1885-1891, XP002540794 ISSN: 0095-1137
- BLOOMQUIST BRIAN T ET AL: "Rapid isolation of flanking genomic DNA using biotin-RAGE, a variation of single-sided polymerase chain reaction" DNA AND CELL BIOLOGY, vol. 11, no. 10, 1992, pages 791-797, XP008109602 ISSN: 1044-5498
- SHIN CHANG HO ET AL: "Detection and typing of HSV-1, HSV-2, CMV and EBV by quadruplex PCR", YONSEI MEDICAL JOURNAL,, vol. 44, no. 6, 30 December 2003 (2003-12-30), pages 1001-1007, XP002531454,

### SEQUENCE LISTING

<110> Genomica S.A.U
<120> Method for detection of Herpesvirus in a test sample
<130> WPP291211
<150> 0806041.0
   <151> 2008-04-03
<160> 24
<170> PatentIn version 3.3
<210> 1
   <211> 23
   <212> DNA
   <213> Artificial
<220>
   <223> HSV amplification primer
<400> 1
   cgcatcatct acggggacac gga 23
<210> 2
   <211> 23
   <212> DNA
   <213> Artificial
<220>
   <223> HSV amplification primer
<400> 2
   atgacgccga tgtacttttt ctt 23
<210> 3
   <211> 18
   <212> DNA
   <213> Artificial
<220>
   <223> HSV-1 amplification primer
<400> 3
   ccttcgaaca gctcctgg 18
<210> 4
   <211> 20
   <212> DNA
   <213> Artificial
<220>
   <223> HSV-2 amplification primer
<400> 4
   tccattttcg ttttgtgccg 20
<210> 5
   <211> 23
   <212> DNA
   <213> Artificial
<220>
   <223> HHV-6 amplification primer
<400> 5
   gaggtaattt atggtgatac gga 23
<210> 6
   <211> 23
   <212> DNA
   <213> Artificial
<220>
   <223> HHV-6 amplification primer
<400> 6
   tgtctaccaa tgtatctttt ttt 23
<210> 7
   <211> 20
   <212> DNA
   <213> Artificial
<220>
   <223> HHV-6 amplification primer
<400> 7
   ggcgacttga acagacgatc 20
<210> 8
   <211> 20
   <212> DNA
   <213> Artificial
<220>
   <223> HHV-6 amplification primer
<400> 8
   ggcgatttga acaagcgatc 20
<210> 9
   <211> 23
   <212> DNA
   <213> Artificial
<220>
   <223> VZV amplification primer
<400> 9
   aaggttatat atggagatac gga 23
<210> 10
   <211> 23
   <212> DNA
   <213> Artificial
<220>
   <223> VZV amplification primer
<400> 10
   attaccccaa tgtacttttt ctt 23
<210> 11
   <211> 23
   <212> DNA
   <213> Artificial
<220>
   <223> CMV amplification primer
<400> 11
   cgggtcatct acggggacac gga 23
<210> 12
   <211> 21
   <212> DNA
   <213> Artificial
<220>
   <223> CMV amplification primer
<400> 12
   aagccgtcac gtagtgcgcc a 21
<210> 13
   <211> 18
   <212> DNA
   <213> Artificial
<220>
   <223> EBV amplification primer
<400> 13
   cagggagatg ggggccac 18
<210> 14
   <211> 20
   <212> DNA
   <213> Artificial
<220>
   <223> HV7-FW amplification primer
<400> 14
   aggtccaaca tgcacagtga 20
<210> 15
   <211> 20
   <212> DNA
   <213> Artificial
<220>
   <223> HV7-RW amplification primer
<400> 15
   ggcaaagaaa atgtgggcta 20
<210> 16
   <211> 19
   <212> DNA
   <213> Artificial
<220>
   <223> HV8S amplification primer
<400> 16
   ggtcatatac ggcgacact 19
<210> 17
   <211> 21
   <212> DNA
   <213> Artificial
<220>
   <223> HV8AS amplification primer
<400> 17
   agtaccccca cgtatctctt t 21
<210> 18
   <211> 23
   <212> DNA
   <213> Artificial
<220>
   <223> HER 1 amplification primer
<400> 18
   ctccggcccc tgaatryggc taa 23
<210> 19
   <211> 23
   <212> DNA
   <213> Artificial
<220>
   <223> HER4 amplification primer
<220>
   <221> misc feature
   <222> (19)..(19)
   <223> n = i = inosine
<400> 19
   ctgtgttgaw acytgagcnc cca 23
<210> 20
   <211> 22
   <212> DNA
   <213> Artificial
<220>
   <223> RTS amplification primer
<400> 20
   gcttgggcgt gtctcaaaat ct 22
<210> 21
   <211> 22
   <212> DNA
   <213> Artificial
<220>
   <223> RTA amolification primer
<400> 21
   gtcgccacgg ttgatgagag ct 22
<210> 22
   <211> 28
   <212> DNA
   <213> Artificial
<220>
   <223> Probe CI 1 5'
<400> 22
   cagctggcac gacaggtttc ccgactgg 28
<210> 23
   <211> 23
   <212> DNA
   <213> Artificial
<220>
   <223> CI 1 3' probe
<400> 23
   ttgaagtggt ggcctaacta cgg 23
<210> 24
   <211> 22
   <212> DNA
   <213> Artificial
<220>
   <223> CI 2 5' probe
<400> 24
   cgttccactg agcgtcagac cc 22

## Description

### FIELD OF THE INVENTION

The present invention relates to a method and kit for detection and identification of Herpesvirus in a test sample, in particular of HSV-1, HSV-2 and vzv Optionally, HHV-6 may also be detected in the sample, while also described herein is a method and kit to further allow efficient detection of VZV, CMV, EBV, Enterovirus, HHV-7 and HHV-8.

Particularly advantageous combinations of amplification primers are provided.

Also described herein is a method and kit, for the detection and identification, if present in a test sample, of one or more viral agents selected from the group comprising HSV-1, HSV-2, HHV-6, VZV, CMV, EBV, Enterovirus, HHV-7 and HHV-8.

### BACKGROUND OF THE INVENTION

There are more than 100 known herpesviruses in the family of *Herpesviridae.* Of these, eight are known to infect humans: Herpes simplex virus 1 (HSV-1), herpes simplex virus 2 (HSV-2), varicella-zoster virus (VZV), Epstein- Barr virus (EBV), cytomegalovirus (CMV), herpesvirus 6 (HHV-6), herpesvirus 7 (HHV-7), and herpesvirus 8 (HHV-8).

Herpesviruses are infectious agents which, after an initial acute infection, establish a latent infection, producing, under specific conditions, periodic recurrences. Recurrence of infection with herpesviruses is associated with neurologic pathology and alteration. The most common clinical manifestations of the infections of the central nervous system associated with this group of viruses are aseptic meningitis, encephalitis, meningoencephalitis and polyradiculitis.

Further to neurologic infections produced by herpesviruses, other neurologic infections of viral origin are those produced by viruses of the enterovirus group, as described in EP0789081. Both enteroviruses and herpesviruses are capable of producing neurological syndromes, both in immunocompetent as well as in immunodepressed patients. Therefore, there is a great interest in establishing an effective identification of the agent associated with neurological symptoms.

General laboratory techniques for viral detection include isolation of viruses in cultured cells, serological techniques, as well as detection of genetic material by PCR. The two former techniques are slow and of low sensitivity, thereby focussing recent efforts in the latter technique.

Herpesviruses are enveloped viruses whose genome is a double-stranded DNA molecule, while enteroviruses are un-enveloped viruses whose genome is constituted by a single-stranded RNA molecule of positive polarity.

Some examples of multiplex PCR assays for Herpesvirus detection are described in WO93/25707, WO2004/016219 and US2007/0207453.

WO 2004/016219 describes a method for the detection and identification of one or more human herpesvirus in a sample, wherein the method comprises the use of consensus primers to the conserved herpesvirus DNA polymerase gene sequence. Detection and identification is further carried out using heteroduplex mobility shift assay (HMSA), dot blot assay or real time PCR.

Further, EP0789081 describes a method to detect some viruses belonging to the Herpesviridae family (HSV-1, HSV-2, VZV, CMV, HHV-6 and EBV), as well as Enterovirus.

EP0789081 describes mixtures of reaction primers that are used for enzymatic amplification of the genomes of viruses belonging to the *Herpesviridae* family, such as HSV-1, HSV-2, VZV, CMV, HHV-6, or EBV, as well as *Enterovirus.* The primers are used in a Multiplex reaction which generates amplification fragments of the same size, from which identification of the infectious agent present in a sample can only be performed by means of a second amplification reaction over the products resulting from the first amplification reaction, or else, by hybridization of the amplified sequences with specific oligonucleotide probes.

WO 2007/056463 describes methods and compositions for use in the quantitative and simultaneous analysis of two or more viral, bacterial or protozoan pathogens in a sample. The methods of this invention involve the addition of oligonucleotide primers specific for each pathogen to be detected, together with nucleic acid competitor molecules that are amplified with the pathogen-specific primers at a similar efficiency. Markoulatos P et al. (2000, Journal of Clinical Laboratory Analysis, Vol. 14, N°. 5, pages 214-219), describes a method for the simultaneous detection of HSV-1, HSV-2 and VZV in a biological sample by multiplex PCR. Similarly, Shin CH et al. (2003, Yonsei Medical Journal, Vol. 44, N°. 6, pages 1001-7), describes a method for the simultaneous detection of HSV-1, HSV-2, CMV and EBV in a sample also by multiplex PCR.

US 2007/0141559 describes a method for detecting and differentiating between HSV-1 and HSV-2 in a sample using primer sequences specific for HSV-1 or HSV-2 by multiplex or independent PCRs.

Aspects of the present invention further relate to methods for reducing detection of non-specific cross hybridisation of amplified target-specific nucleic acid sequences; particularly, but not exclusively, in the performance of a detection method as described above.

WO 03/033735 describes a dipping test strip assay system comprising dried reagents which can be used to detect the presence of specific nucleic acid sequence within a sample.

WO 2007/032748 describes a method for detecting methylation in a DNA sample comprising chemically converting unmethylated cytosine residues to uracil, amplifying the chemically converted DNA using primers in which the upper primer is biotinylated and hybridising the labelled product to an oligonucleotide probe together with the addition of strepavidin.

WO99/25867 describes a method for detecting nucleic acids in several samples comprising, preparing the nucleic acids in the sample, removing amplification inhibitors, generating PCR products of sections of the nucleic acid to be detected and automatically detecting the amplification products.

US2005/0287549 describes a method for the simultaneous detection of a number of SNPs. In particular, the use of a primer pair in which one primer is biotinylated is described. The amplification product is then immobilised through avidin-biotin reactions.

EP0795612 describes a method for amplifying and detecting a target nucleic acid in a sample including a post amplification incubation step prior to detection to inactivate the amplification enzymes. A biotinylated amplified product can then be detected by hybridisation with a strepavidin-enzyme conjugate.

WO 01/094638 describes a method of nucleic acid amplification including a thermal cycling protocol, and the use of labelled forward primers in the amplification step.

EP1595960 describes a method for the selective detection of nucleic acid in a sample, comprising amplifying the nucleic acid with two primers, one of which is biotinylated, and hybridising the labelled product to a capture probe.

KR060015668 describes a method for the detection of pathogenic microorganisms comprising amplifying the target sequence with a labelled primer and hybridising the labelled product with probes immobilised to the surface of a DNA chip.

Database Geneseq (Varicella-zoster virus genomic amplification primer) (Database Accession numbers AAT37529 and AAT37524) describe the use of at least one amplification primers in AAT37520-T37522 and in AAT37523-T37532 for the amplification of enterovirus and herpesvirus sequences.

Database Geneseq (Human herpes virus 2-specific probe SEQ ID NO: 5) describes a herpes-virus gene chip comprising a herpes-virus specific probe.

US 2004/110195 describes PCR methods capable of identifying all eight human herpesvirus, including EBV and HHV-6 subtypes using amplification of the herpesvirus DNA polymerase gene. US 2004/110195 also describes amplification of both HSV-1 and HSV-2 with a single pair of primers.

Database Geneseq (Herpes simplex virus genomic amplification primer) describes the use of at least one primer in AAT37520-T37522 and in AAT37523-T37532 for the amplification of enterovirus and herpesvirus sequences.

Markoulatos Panayotis et al. (J. Clinical Microbiology) describes a multiplex PCR assay for single tube amplification of HSV-1, HSV-2, VZV, CMV and EBV followed by visualisation of the amplified products by agarose gel electrophoresis.

### SUMMARY OF THE INVENTION

The problem to be solved by the present invention is the provision of a method that allows improved detection of HSV-1, HSV-2 and vzv over prior art methods.

The envisaged method should desirably further allow detection of HHV-6, CMV, EBV and enterovirus.

This has been solved by the design of amplification primers that, when used in amplification reactions according to the present invention, result in an improved sensitivity of detection.

Use of the newly designed primers is compatible with at least as sensitive detection levels of VZV, CMV, EBV and enterovirus, as those of prior art methods. Also described herein, are primers for an efficient amplification and detection of HHV-7 and HHV-8.

The present invention provides pairs of primers which are not simple alternatives to already known primers of the state of art, but that result in more efficient amplification levels.

An advantage of the method according to the present invention is that the efficiency of the amplification primers is not negatively affected by the presence of the additional pairs of amplification primers:
- W1S and W1AS (for amplification of virus VZV), in the case of HSV-1 and HSV-2 amplification method, or
- CMVS and CMVAS-21 (for amplification of virus CMV), EBVAS-23 and CMVS (for amplification of virus EBV), HER1 and HER4 (for amplification of enterovirus), HV7-FW and HV7-RW (for amplification of virus HHV-7), and HV8S and HV8AS (for amplification of virus HHV-8), in the case of HHV-6 amplification method.

The difficulties in designing successful multiplex PCRs are widely recognised by those skilled in the art (see for instance Markoulatos et al., 2000, Journal of Clinical Laboratory Analysis, Vol. 14, N°. 5, pages 214-219; or Shin et al., 2003, Yonsei Medical Journal, Vol. 44, N°. 6, pages 1001-7). It will therefore be recognised that design of primers for use in such multiplex reactions is not a routine undertaking.

A further advantage of the present invention is that amplification with the amplification primers of the present invention results in amplification products of different sizes, thereby allowing a user to distinguish between the different viruses of interest without the requirement of additional steps, such as a second amplification reaction of the products resulting from the first amplification reaction, or hybridisation of the amplified sequences with specific oligonucleotide probes.

### BRIEF DESCRIPTION OF THE DRAWINGS

Figure 1. Agarose gel electrophoresis corresponding to amplification fragments of Example 1.
Figure 2. Agarose gel electrophoresis corresponding to amplification fragments of Example 2.
Figure 3. Agarose gel electrophoresis corresponding to amplification fragments obtained under experimental conditions of Example 2.
Figure 4. List of amplification primers.
Figure 5. Comparison of the results obtained, either without or with selective primer biotinylation, and in the presence of an internal control, for HSV-1 and HSV-2. Panel A: Without selective biotinylation of the primers; Panel B: With selective biotinylation of the primers.
Figure 6. Comparison of the results obtained, either without or with selective primer biotinylation, and in the presence of an internal control, for CMV and EBV. Panel A: Without selective biotinylation of the primers; Panel B: With selective biotinylation of the primers.
Figure 7. Illustration of probe labelling technique for reduction of detection of cross-hybridisation.

### DETAILED DESCRIPTION OF THE INVENTION

Definitions. The following terms have the indicated meanings in the specification unless expressly indicated to have a different meaning:
- Amplification Primers: Nucleic acids that bind to and allow amplification of one or more target sequences.
- Array tube: An individual array vessel which has a shape and size typical of a laboratory reaction vessel (for example, a 1.5 ml Eppendorf tube) with a microarray arranged therein in which microarray based tests can be carried out.
- Array vessel: A reaction vessel with flat bottom comprising a microarray. The probe molecules of the microarray may be printed on a solid support wherein this solid support can be the bottom of an array vessel, or a different solid support attached to the bottom of an array vessel.
- Microarray: Arrangement of molecular probes on a surface, wherein the position of each probe is separately determined.
- Multiplex PCR and RT-PCR reactions: PCR and RT-PCR reactions that allow amplification of two or more nucleic acid sequences if present.
- Probes: Nucleic acid with ability to specifically bind to a target nucleic acid sequence. This includes DNA, RNA, PNA, and any other form or modification.
- Sensitivity of detection: Minimum number of copies detected. "Improved sensitivity" here means that there is a lower minimum detectable copy number.
- Strip of vessels: A set of array vessels, usually 8, each with a microarray arranged therein, in which microarray based tests can be carried out.
- Target sequences: Sequences to be detected.
- Target-specific probes: Probes that hybridize specifically with the target sequences amplifiable in the amplification reactions.

### Original Amplification primers for detection of HSV-1 and HSV-2:

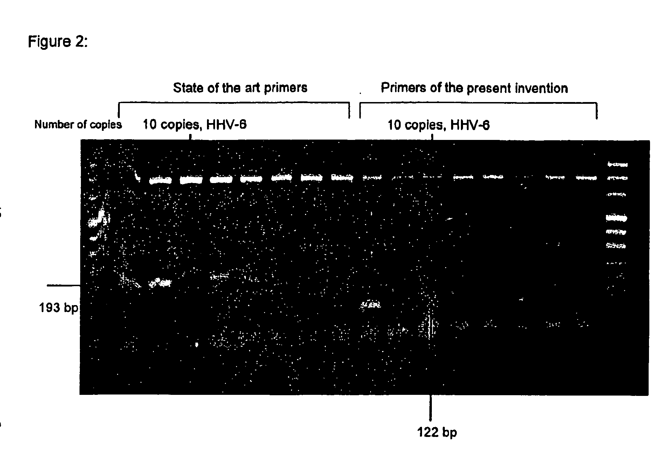

SEQ ID N° 1 (CGCATCATCTACGGGGACACGGA) and SEQ ID N° 2 (ATGACGCCGATGTACTTTTTCTT) (These are referred to in EP0789081 as SEQ ID N° 4 and 9, respectively; See Figure 2 and Example 1 of EP0789081).

The amplification product was common to HSV-1 and HSV-2.

In order to distinguish between them, a second amplification of the amplification product had to be carried out, as in Example 9 of EP0789081.

### Amplification conditions, for detection of HSV-1 and HSV-2, according to the present invention:

- Amplification of HSV-1: Newly designed primer of SEQ ID N° 3, HSV1S-18 (CCTTCGAACAGCTCCTGG), and SEQ ID N° 2 primer HSV1AS (ATGACGCCGATGTACTTTTTCTT), referred to as SEQ ID N° 9 primer in EP0789081.
- Amplification of HSV-2: Newly designed primer of SEQ ID N° 4, HSV2S-20 (TCCATTTTCGTTTTGTGCCG) and SEQ ID N° 2 primer HSV1-AS (ATGACGCCGATGTACTTTTTCTT), referred to as SEQ ID N° 9 primer in EP0789081.

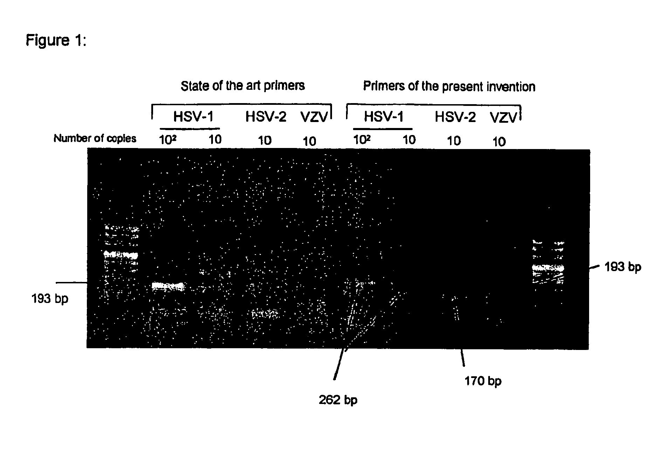

Amplification with the new pairs of amplification primers results in a higher sensitivity of detection when compared with amplification with the original primers, as can be observed in Figure 1 corresponding to Example 1.

Further, amplification with primers HSV1S-18 and HSV1-AS is specific for HSV-1, and produces a fragment of 262 bp in case HSV-1 is present in the sample, while amplification with primers HSV2S-20 and HSV1-AS is specific for HSV-2, and produces a fragment of 170 bp in case HSV-2 is present in the sample.

Products of 262 and 170 bp are distinguishable in Agarose Gel Electrophoresis, thereby avoiding the requirement for further amplification of the amplification product.

### Original Amplification primers for detection of HHV-6:

SEQ ID N° 5 (GAGGTAATTTATGGTGATACGGA) and SEQ ID N° 6 (TGTCTACCAATGTATCTTTTTTT), referred to as SEQ ID N° 7 and 12, respectively, in EP0789081 (see Figure 2 and Example 1 of EP0789081).

### Amplification primers for detection of HHV-6, according to the present invention:

Newly designed primer HHV6A-AS (GGCGACTTGAACAGACGATC), of SEQ ID N° 7, and SEQ ID N° 5 primer, HV6S (GAGGTAATTTATGGTGATACGGA), referred to as SEQ ID N° 7 in EP0789081.

An even further improvement is accomplished when, in the mixture of primers HHV6A-AS and HV6S, 50% of the amount of primer HHV6A-AS is substituted by newly designed primer HHV6B-AS (GGCGATTTGAACAAGCGATC), of SEQ ID N° 8. The reason for the difference is that primer HHV6A-AS specifically amplifies subtype A of HHV-6, while HHV6B-AS specifically amplifies subtype B of HHV-6.

Example 2 and corresponding Figure 2, display the improvement of sensitivity obtained with the new amplification primers, with respect to that obtained with the original mixture of amplification primers of SEQ ID N° 5 and SEQ ID N° 6 (referred to in EP0789081 as SEQ ID N° 7 and SEQ ID N° 12, respectively).

Also described herein are amplification conditions which allow amplification of VZV, CMV, EBV and enterovirus in at least as sensitive detection levels as those of prior art methods. Further, also described herein are primers for an efficient amplification and detection of HHV-7 and HHV-8. Some representative images are provided within Figures 1 to 3, corresponding to Examples 1 and 2 below.

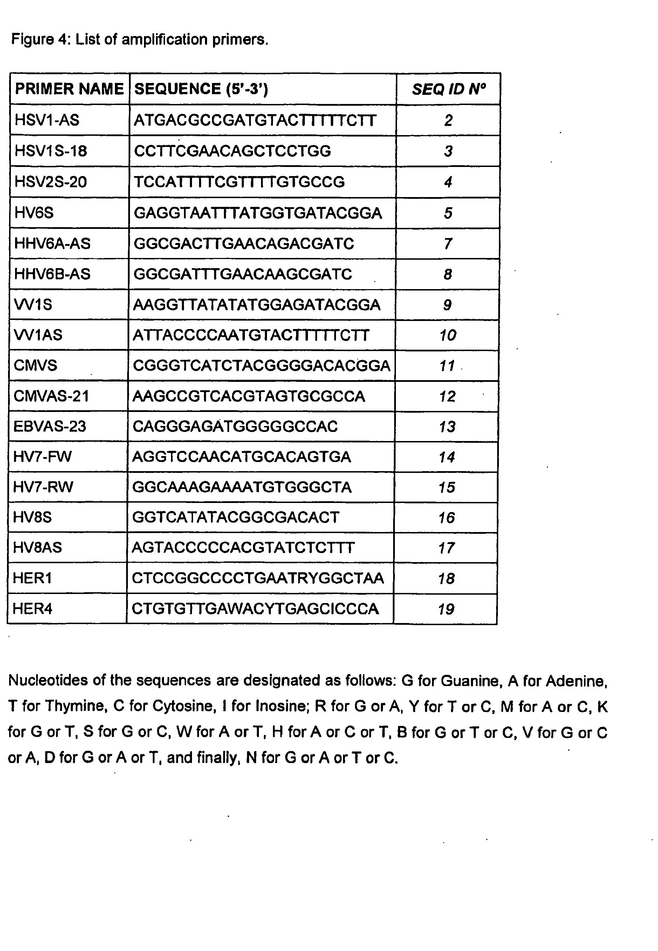

In a preferred embodiment of the present invention, the amplification primers correspond to those of Figure 4.

The primers for amplification of Enterovirus allow amplification of the different types of Enteroviruses: Echovirus, Poliovirus and Cosackievirus, if present in the sample.

In a preferred aspect, the method to detect HSV-1, HSV-2, HHV-6, VZV, CMV, EBV, HHV-7, HHV-8 and enterovirus comprises amplification of viral genetic material in two independent reactions:
One corresponding to HSV-1, HSV-2 and VZV (tube 1), and another corresponding to HHV-6, CMV, EBV, HHV-7, HHV-8 and enterovirus (tube 2), wherein the corresponding respective primers are incubated with the sample of interest. In a preferred aspect, the reaction of tube 1 is a PCR, and that of tube 2 is a RT-PCR.

In a preferred aspect of the present invention, tubes 1 and 2 comprise the following components:

### Tube 1:

| **Multiplex PCR 1 Reactants** | **Volume (µl)** |
|---|---|
| Sample | 5 |
| DNA Polymerase 10X Buffer | 5 |
| MgCl₂, 25 mM | 2 |
| dNTPs, 10 mM | 1 |
| HSV1S-18, 0.1µg/µl | 0.8 |
| HSV1-AS, 0.1µg/µl | 1.6 |
| HSV2S-20, 0.1µg/µl | 0.8 |
| W1S, 0.1µg/µl | 0.8 |
| W1AS, 0.1µg/µl | 0.8 |
| DNA Polymerase | 0.8 |
| Nuclease Free Water | until final volume 50 µl |

In a preferred embodiment, the pair of primers used for amplification of HSV-1 is formed by HSV1S-18 and HSV1-AS; The pair of primers for HSV-2 is formed by HSV2S-20 and HSV1-AS; And the pair of primers for VZV is: W1S (AAGGTTATATATGGAGATACGGA), SEQ ID N° 9, and W1AS (ATTACCCCAATGTACTTTTTCTT), SEQ ID N° 10. (These latter primers correspond, respectively, to those of SEQ ID N° 5 and 10 of Figure 2 of EP0789081).

The expected sizes of the corresponding amplification primers are:

| **Virus** | **Size (bp)** |
|---|---|
| HSV-1 | 262 |
| HSV-2 | 170 |
| VZV | 193 |

### Tube 2:

| **Multiplex RT-PCR 2 Reactants** | **Volume (µl)** |
|---|---|
| Sample | 5 |
| RT-PCR 5x buffer | 10 |
| dNTPs, 10 mM | 2 |
| HV6S, 0.1µg/µl | 0.8 |
| HHV6A-AS + HHV6B-AS, 0.1µg/µl (50% each) | 0.8 |
| CMVS, 0.1 µg/µl | 1.6 |
| CMVAS-21, 0.1µg/µl | 0.8 |
| EBVAS-23, 0.1µg/µl | 0.8 |
| HV7-FW, 0.1µg/µl | 0.8 |
| HV7-RW, 0.1µg/µl | 0.8 |
| HV8S, 0.1µg/µl | 0.7 |
| HV8AS, 0.1µg/µl | 0.7 |
| HER1, 20 µM | 0.4 |
| HER4, 40 µM | 0.2 |
| Nuclease Free Water | until final volume 50 µl |

In a preferred aspect, the pair of primers used for amplification of HHV-6 is formed by HV6S (SEQ ID N° 5, referred to as SEQ ID N° 7 in Figure 2 of EP0789081) and a mixture of primers HHV6A-AS and HHV6B-AS, SEQ ID N° 7 and 8, respectively, (see above and Figure 4 for the corresponding sequences); The pair of primers for CMV is formed by SEQ ID N° 11, CMVS (corresponding to SEQ ID N° 6 of Figure 2 of EP0789081) and SEQ ID N° 12, CMVAS-21 (See Figure 4); The pair of primers for EBV is: SEQ ID N° 13, EBVAS-23 (See Figure 4) and SEQ ID N° 11, CMVS (corresponding to SEQ ID N° 6 of Figure 2 of EP0789081); Remaining pairs of primers are: HV7-FW and HV7-RW, for HHV-7; HV8S and HV8AS for HHV-8; HER1 and HER4 for enterovirus (see Figure 4).

The expected sizes of the corresponding amplification primers are:

| **Virus** | **Size(bp)** |
|---|---|
| HHV-6 | 122 |
| CMV | 106 |
| EBV | 132 |
| HHV-7 | 207 |
| HHV-8 | 192 |
| Enterovirus | 328 |

In a preferred aspect, the PCR reactions of Tube 1 and 2 are run in a thermocycler programmed with the following cycling profile:

| | |
|---|---|
| 1 cycle | 45°C 45 min |
| 1 cycle | 95°C 15 min |
| | |
| 45 cycles | 95°C 30 sec |
| | 56°C 1 min 30 sec |
| | 72°C 1 min |
| | |
| 1 cycle | 72°C 10 min |

The differences of the fragments' sizes allow to distinguish which virus is present in the sample, without further needing to carry out a second amplification reaction.

Further, sensitivity values obtained with the method according to the present invention are at least the same as those obtained with the pairs of primers of the state of the art (see Examples 1 and 2, as well as Figures 1 to 3).

In another preferred embodiment, one or both reaction tubes further comprise internal controls. In a preferred embodiment, the protocol of the present invention includes an internal control of the nucleic acids extraction step. In another preferred embodiment, the protocol of the present invention includes an internal control of the nucleic acid amplification step.

A preferred internal control would be a DNA plasmid that would be amplifiable with amplification primers RTS (GCTTGGGCGTGTCTCAAAATCT, SEQ ID NO 20) and RTA (GTCGCCACGGTTGATGAGAGCT, SEQ ID NO 21). Preferably, the internal control would be:

| **Multiplex Reactants** | **Volume (µl)** |
|---|---|
| internal Control DNA, 10³ copies | 5 |
| RTS 20 µM | 0.5 |
| RTA 20µM | 0.5 |

In a final reaction volume of 50 µl.

The amplification product corresponding to the internal control DNA is a DNA fragment of 885 bp.

Further preferred internal controls described herein would be an RNA fragment that would preferably be added to the test sample prior to the nucleic acid extraction. Another preferred internal control would be an RNA fragment that would preferably be added to the nucleic acids after extraction from the test sample. In a more preferred aspect, the RNA fragment would be present within the vessel containing the amplification reactants, prior to incubation with the nucleic acids obtained from the test sample.

In a more preferred aspect, the RNA fragment could be obtained by RNA transcription of a plasmid. In another preferred aspect the RNA fragment could be obtained by chemical synthesis.

An inherent problem of multiplex PCRs is that the presence of several primers in the same amplification reaction could lead to an interaction among the primers present, that might preclude their hybridisation with their target sequences and corresponding amplification. This technical hurdle was overcome by the specific combination of amplification primers of the Multiplexes corresponding to Tube 1 and Tube 2.

In a preferred embodiment, types of samples that can be processed by the present invention are swabs, paraffin-embedded biopsies, as well as saline, plasma, and cerebral spinal fluid.

In another preferred embodiment, extraction of genetic material can be carried out both by automatic as well as by manual extraction techniques of the state of the art.

In an even more preferred embodiment, an automatic extraction system that can be used for the isolation of both DNA and RNA of the samples of the present invention is the NucliSENS easyMAG of BioMerieux (EP1694813), which uses magnetic particles in combination with BioMerieux's BOOM® technology for universal isolation of total nucleic acid from a wide range of sample volumes and types.

The skilled person will be aware of techniques and methods for manual processing of samples to extract DNA and other nucleic acids; and any such suitable method may be used.

In a particular example, nucleic acids are extracted from 50 µl of test sample. After the extraction step, a precipitate is resuspended in 25 µl RNase-free water.

In a preferred/ particular embodiment, 5 µl of the 25 µl comprising the genetic material extracted from the test sample, are added to the vessel containing the amplification reactants, in a final volume of 50 µl.

In a preferred embodiment of the present invention, the PCR products obtained with the amplification primers according to the present invention, can be further characterized by means of microarray technology. The microarray technology provides the simultaneous detection of multiple molecular markers for diagnostic use, while providing the controls needed to ensure the reliability of the results.

In a preferred embodiment of the present invention, a label may be introduced in the amplified DNA during its amplification to allow further detection, preferably a label that provides a signal that may be detected by colorimetric methods. In the most preferred embodiment, the label is biotin. However, any other kind of label known in the art may be used (e. g. digoxigenin). Radioactive labels may be used, or fluorophores, in certain embodiments. In a preferred embodiment, labelling of amplified DNA may be achieved by adding modified nucleotides bearing a label (e. g. biotinylated or digoxigenin dUTP derivatives) in the amplification mixture. In another even more preferred embodiment, the label is contained in the amplification primers.

In a preferred embodiment of the present invention, amplified DNA, previously denatured, is hybridized with target-specific probes to minimise the problem of multiplex PCRs which consists in the obtention of aspecific amplification fragments, due to the presence of several primers.

In a preferred embodiment, denaturing of amplified DNA can be performed by heating. Other ways to prepare single stranded DNA after amplification may be used as well; for example, chemical means.

The primers of the present invention, labelled with biotin, were tested in order to check their amplification efficiency by agarose gel electrophoresis. Labelling of either one or two of each pair of primers was tested. The conditions selected were as follows:

**X indicates condition chosen.**

| | HSV-1 | HSV-2 | VZV | CMV | EBV | HHV-6 | HHV-7 | HHV-8 | Entero |
|---|---|---|---|---|---|---|---|---|---|
| 1 primer labelled | | | | | | X | | | X |
| 2 primers labelled | X | X | X | X | X | | X | X | |

In general, labelling of one or two of the primers with biotin did not affect the amplification efficiency of the primers. In most cases, labelling of both the primers was selected.

Nothwithstanding this, when amplification products were denatured and hybridized with probes that were specific for each virus, there were problems of cross-hybridization (i.e. of binding with probes specific for another virus): Thus, the amplification product of HSV-1 cross-hybridized with the probe specific for HSV-2; and the amplification product of EBV cross-hybridized with the probe specific for CMV. This problem was not to be easily solved due to the sequence homology between the viruses.

The problem was solved in the following way: Only one primer of each pair of amplification primers of the viruses that cross-hybridize (i.e. HSV-1 or EBV), was biotin-labelled. Thereby, the nucleic acid strand of the amplification fragments that hybridizes with the probe specific for another virus (i.e., cross-hybridizes) is not labelled, and therefore it does not produce any signal.

In this way, even though the complementary strand hybridizes with the probe corresponding to the other virus, no signal will be generated.

In the present case, the strand of HSV-1 that cross-hybridizes with HSV-2 was not labelled, nor was the EBV strand that cross-hybridizes with CMV. Specifically, for amplification of HSV-1, HSV1S-18 was unlabelled and HSV1-AS was biotin-labelled. For amplification of EBV, EBVAS-23 was unlabelled and CMVS was biotin-labelled. Both primers for amplification of HSV-2 (HSV1-AS and HV2S-20) and of CMV (CMVS and CMVAS-21) were biotin-labelled.

Any labelling method might be used.

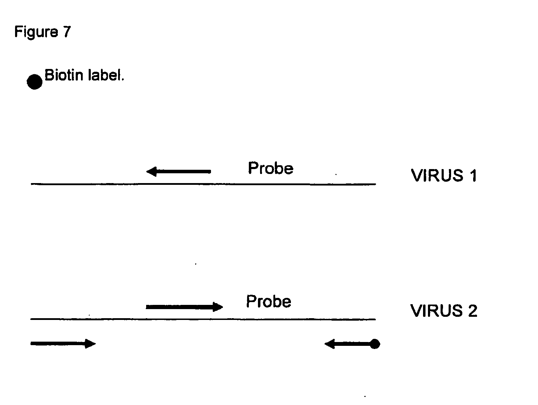

An illustration of the labelling scheme used is given in Figure 7.

Adequate amplification conditions of the Multiplex PCR 1 and Multiplex RT-PCR 2 reactions are as follows:

| Multiplex PCR 1 Reactants | Volume (µl) |
|---|---|
| Sample | 5 |
| DNA Polymerase 10X Buffer | 5 |
| MgCl₂, 25 mM | 2 |
| dNTPs, 10 mM | 1 |
| HSV1S-18, 0.1µg/µl | 0.8 |
| B- HSV1-AS, 0.1µg/µl | 1.6 |
| B- HSV2S-20, 0.1µg/µl | 0.8 |
| B- VV1S, 0.1µg/µl | 0.8 |
| B- VV1AS, 0.1µg/µl | 0.8 |
| DNA Polymerase | 0.8 |
| Nuclease Free Water | until final volume 50 µl |

| Multiplex RT-PCR 2 Reactants | Volume (µl) |
|---|---|
| Sample | 5 |
| RT-PCR 5x buffer | 10 |
| dNTPs, 10 mM | 2 |
| HV6S, 0.1µg/µl | 0.8 |
| B- HHV6A-AS + B- HHV6B-AS, 0.1µg/µl (50% each) | 0.8 |
| B- CMVS, 0.1µg/µl | 1.6 |
| B- CMVAS-21, 0.1µg/µl | 0.8 |
| EBVAS-23, 0.1µg/µl | 0.8 |
| B- HV7-FW, 0.1µg/µl | 0.8 |
| B- HV7-RW, 0.1µg/µl | 0.8 |
| B- HV8S, 0.1µg/µl | 0.7 |
| B- HV8AS, 0.1µg/µl | 0.7 |
| B- HER1, 20 µM | 0.4 |
| HER4, 40 µM | 0.2 |
| Nuclease Free Water | until final volume 50 µl |

Preferably, one or both of the Multiplex reactions further comprise an Internal Control DNA, for instance in the form of a DNA plasmid, and primers for its amplification. For instance:

| Reactants | Volume (µl) |
|---|---|
| Internal Control DNA, 10³ copies | 5 |
| RTS 20 µM | 0.5 |
| RTA 20µM | 0.5 |

Introduction of above mentioned components in either Multiplex reaction does not affect the result obtained.

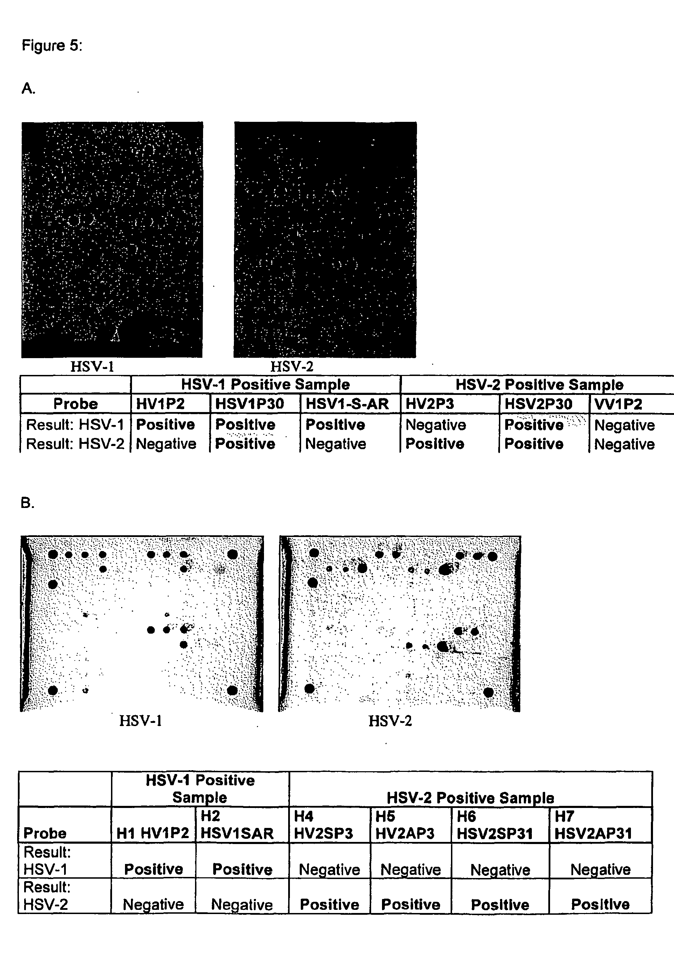

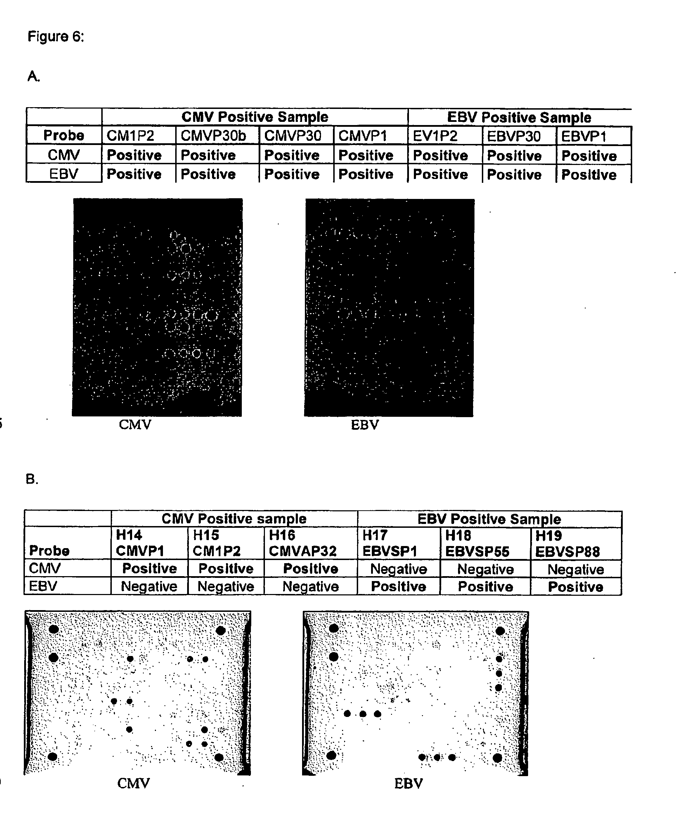

Comparison of the results obtained under above-mentioned preferred conditions, either without or with selective primer biotinylation, and in the presence of above-mentioned internal control, are displayed, for HSV-1 / HSV-2 and for CMV / EBV, respectively, in Figures 5 and 6.

The amplified fragments obtained were hybridised in an array tube with different selective probes corresponding to each virus, as indicated.

In a preferred embodiment of the present invention, the single stranded DNA is incubated with a plurality of target-specific probes provided on a microarray. At least one, but preferably more than one probe with ability to hybridise with each target sequence, are provided on the microarray. In certain embodiments of the invention, the single stranded DNA may be incubated with target-specific probes provided in solution; however, it is preferred that the probes are provided in a solid support.

In a preferred embodiment of the present invention, the probes are contained in a microarray, which may be placed on a slide or contained in a reaction vessel, which is then called an array vessel. Array vessels may have different formats of presentation, including individual array vessels, in particular, array tubes, or sets of array vessels arranged in strips or flat plates. Usually, plates consist of sets of strips of array vessels. Thus, a microarray of the present invention may be contained in an individual array vessel. Alternatively, two or more microarrays may be contained in a strip of vessels. In a preferred embodiment, the strip of vessels is constituted by 8 vessels. Further, three or more array vessels may be arranged in a set of strip of vessels. In another preferred embodiment, the set of strip of vessels is a microtiter plate.

In preferred embodiments, the probe molecules of the microarray may be printed on a solid support wherein this solid support can be the bottom of an array vessel or a different solid support attached to the bottom of an array vessel. This means that the surface of the microarray may be the flat bottom of the array vessel. Alternatively, the surface of the microarray may be a solid support attached to the bottom of the array vessel.

In an embodiment of the present invention, the reaction vessel has a typical size for a laboratory reaction vessel. Typical filling volumes lie in the range of 100 µl to 2.5 ml, but can also be higher or lower in special embodiments. Especially preferably the reaction vessel is an array tube. i.e. an array vessel with a normal filling volume for a standard Eppendorf tube of up to 1.5 ml. Further preferred filling volumes are up to 0.4 ml, up to 0.5 ml, up to 0.7 ml, up to 1.0 ml or up to 2.0 ml.

Due to the labelling of the amplified DNA, wherever sample molecules interact with probe molecules on the surface of the microarray, a reporter reagent binds the label and produces visible signals which may be detected by a detection device. The interacting probe and sample molecules are identified by the location of the signal on the surface of the microarray. In the particular case where sample DNA molecules are labelled with biotin, the reporter agent can be horseradish peroxidase covalently joined to streptavidin. The latter binds specifically to biotin, and the peroxidase triggers the precipitation of substrates like tetramethylbenzidine (TMB). Any other reaction that results in a precipitate on array elements, and that can be used to detect the interaction between target and probe molecules according to the present invention may be used. The probes of the present invention can be obtained by different methods, such as chemical synthesis (e. g. by the conventional phosphotriester method) or genetic engineering techniques, for example by molecular cloning of recombinant plasmids in which corresponding nucleotide sequences have been inserted and can be latter obtained by digestion with nucleases.

Specific probes may be designed using the nucleic acid alignment program Oligo 6. The parameters of Tm and G/C ratio are analysed in all cases, and secondary-structure formation was avoided. Preferred probes of the present invention are those with the same Tm under the salt concentration which is used in the hybridisation step. In a preferred embodiment, probes are selected to bind to their corresponding target sequences under the same hybridisation conditions. The skilled person will be aware of other ways in which specific probes may be designed.

In a particular embodiment of the present invention, the probes are selected so that they do not hybridize with genomic DNA, and do not hybridize in an unspecific way with amplified fragments corresponding to other viruses.

In a preferred embodiment, one or more probes of the present invention are provided on a solid support.

In another preferred embodiment, two or more target-specific probes for the same target sequence are provided on the solid support, in order to improve detection of the viral agents of interest.

In a preferred embodiment, the probes of the present invention are of 30 nt in length or less.

Said probes or mixtures of probes may be immobilized in a single location of the solid support, preferably in two distinct locations of the solid support and more preferably in three distinct locations of the solid support.

In a preferred embodiment, the probes of the present invention are provided on a solid support located within an array vessel.

In another embodiment, the probes of the present invention are provided on a solid support located within an array strip.

In a more preferred embodiment, the array strip of the present invention has 8 array vessels.

In one preferred embodiment, the solid support is a coated glass slide.

In another preferred embodiment, the solid support is the bottom of an array vessel, the array vessel being either an individual array tube, or a component of a strip of vessels or set of strip of vessels, such as a microtiter plate.

In a preferred embodiment, the interactions taking plate between DNA amplified with the pairs of primers of the present invention, and corresponding detection probes, take place on an individual array vessel, on a strip of vessels, or on a set of strips of vessels.

In a preferred embodiment, visualization of such interactions consists of the following steps:
■ First, the image of the array is captured using an optical device,
■ Then, the image is analysed,
■ Finally, a report containing an interpretation of the result is provided.

Preferably, the image is analysed by means of appropriate software.

Any device suitable for this processing can be used.

In a particular embodiment, probes for specific detection of the internal control amplifiable with amplification primers RTS and RTA are also present in the microarray. In particular, probes Cl 1 5' (CAGCTGGCACGACAGGTTTCCCGACTGG, SEQ ID NO 22), Cl 1 3' (TTGAAGTGGTGGCCTAACTACGG, SEQ ID NO 23) and Cl 2 5' (CGTTCCACTGAGCGTCAGACCC, SEQ ID NO 24) may be used.

### Examples

The examples provided below merely illustrate the invention and in no way limit the scope of the accompanying claims.

### EXAMPLE 1.

5 µl of the sample to be analysed were added to amplification reactants of Multiplex PCR 1, in a final reaction volume of 50 µl, as follows:

| **Multiplex PCR 1 Reactants** | **Volume (µl)** |
|---|---|
| Sample | 5 |
| Buffer Taq Gold DNA Polymerase 10X | 5 |
| MgCl₂, 25 mM | 2 |
| dNTPs, 10 mM | 1 |
| HSV1S-18, 0.1 µg/µl | 0.8 |
| HSV1-AS, 0.1µg/µl | 1.6 |
| HSV2S-20, 0.1µg/µl | 0.8 |
| VV1S, 0.1µ/µl | 0.8 |
| VV1AS, 0.1µg/µl | 0.8 |
| Internal Control DNA, 10³ copies | 5 |
| RTS 20 µM | 0.5 |
| RTA 20µM | 0.5 |
| Taq Gold DNA Polymerase | 0.8 |
| Nuclease Free Water | until final volume 50 µl |

PCR reactions were run in a thermocycler programmed with the following cycling profile:

| | |
|---|---|
| 1 cycle | 45°C 45 min |
| 1 cycle | 95°C 15 min |
| | |
| 45 cycles | 95°C 30 sec |
| | 56°C 1 min 30 sec |
| | 72°C 1 min |
| | |
| 1 cycle | 72°C 10 min |

The pair of primers formed by the newly designed primer of SEQ ID N° 3 HSV1S-18 (CCTTCGAACAGCTCCTGG) and SEQ ID N° 2, HSV1-AS (ATGACGCCGATGTACTTTTTCTT), the latter referred to as SEQ ID N° 9 primer in EP0789081, produces a fragment of 262 bp which corresponds to HSV-1 (See Figure 1); Further, the pair of primers formed by the newly designed primer of SEQ ID N° 4, HSV2S-20 (TCCATTTTCGTTTTGTGCCG) and SEQ ID N° 2, HSV1-AS (ATGACGCCGATGTACTTTTTCTT), the latter referred to as SEQ ID N° 9 primer in EP0789081, produces a fragment of 170 bp which corresponds to HSV-2.

Alternatively, in a separate reaction, the pair of primers used in EP0789081, formed by SEQ ID N° 1 (CGCATCATCTACGGGGACACGGA), referred to as SEQ ID N° 4 in EP0789081, and SEQ ID N° 2 (ATGACGCCGATGTACTTTTTCTT), referred to as SEQ ID N° 9 in EP0789081, (Figure 2 and Example 1 of EP0789081), was used, under the same conditions as above, instead of HSV1S-18, HSV1-AS and HSV2S-20. A volume of 1.8 µl of a solution of 0.1 µg/µl of each primer, was used. The amplification product of these primers is common to HSV-1 and HSV-2.

The comparative results obtained with the different amplification primers are displayed in Figure 1.

In both reactions, the pair of primers used for amplification of VZV was: SEQ ID N° 9, VV1S (AAGGTTATATATGGAGATACGGA), and SEQ ID N° 10, VV1AS (ATTACCCCAATGTACTTTTTCTT). These primers correspond, respectively, to those of SEQ ID N° 5 and 10 of Figure 2 of EP0789081. The expected size of the corresponding amplification fragment is 193 bp.

As can be observed in Figure 1, the reaction according to the present invention results in improved sensitivity values of detection of HSV-1, HSV-2 and VZV, when compared with the reaction carried out with the primers of the state of the art. In the case of VZV, this may be due to the particular advantageous combination of primers of the reaction according to the present invention. Comparison of sensitivity values obtained is as follows:

| Virus | Sensitivity (HSV1S-18, SEQ HSV2S-20, HSV1-AS) | Sensitivity (HSV1-AS, ID N° 4 (CGCATCATCTACGGGGACACGGA)) |
|---|---|---|
| HSV-I | 10 | 10² |
| HSV-II | 10 | >10 |
| VZV | 10 | >10 |

### EXAMPLE 2.

5 µl of the sample to be analysed were added to amplification reactants of Multiplex RT-PCR 2, in a final volume of 50 µl, as follows:

| Multiplex RT-PCR 2 Reactants | Volume (µl) |
|---|---|
| Sample | 5 |
| 5X QIAGEN OneStep RT-PCR buffer | 10 |
| 5x Q-solution | 10 |
| HV6S, 0.1 µg/µl | 0.8 |
| HHV6A-AS + HHV6B-AS, 0.1 µg/µl (50% each) | 0.8 |
| dNTPs, 10 mM | 2 |
| CMVS, 0.1µg/µl | 1.6 |
| CMVAS-21, 0.1µg/µl | 0.8 |
| EBVAS-23, 0.1µg/µl | 0.8 |
| HV7-FW, 0.1 µg/µl | 0.8 |
| HV7-RW, 0.1µg/µl | 0.8 |
| HV8S, 0.1µg/µl | 0.7 |
| HV8AS, 0.1 µg/µl | 0.7 |
| HER1, 20 µM | 0.4 |
| HER4, 40 µM | 0.2 |
| Internal Control DNA, 10³ copies | 5 |
| RTS 20 µM | 0.5 |
| RTA 20µM | 0.5 |
| QUIAGEN OneStep RT-PCR Enzyme mix | 2 |
| Nuclease Free Water | until final volume 50 µl |

Amplification conditions were the same as for Multiplex PCR 1.

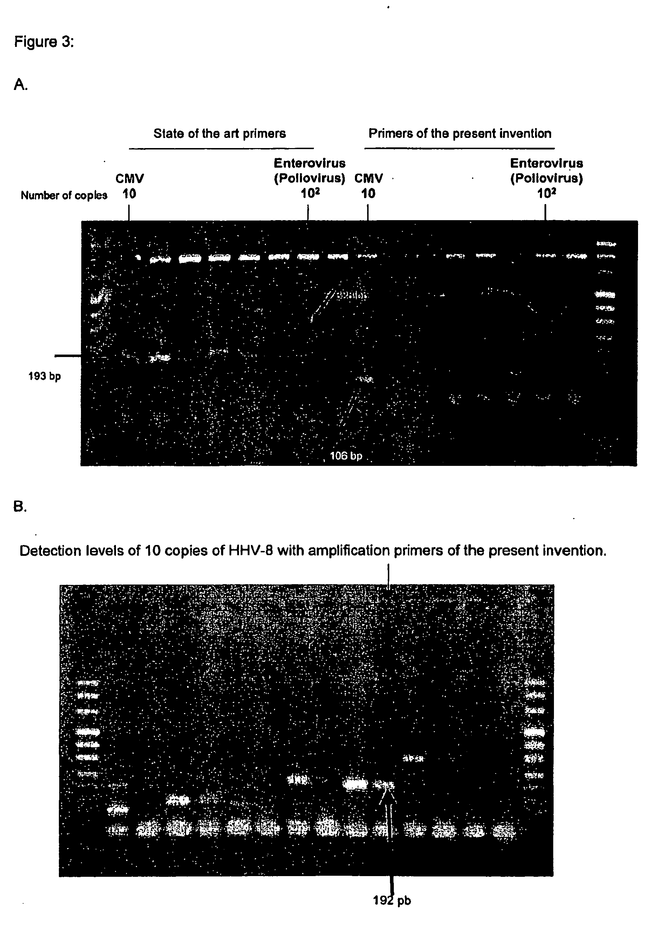

Alternatively, in a separate reaction, the pair of primers described in EP0789081 for HHV-6, CMV, EBV and Enterovirus (see Figure 2 and Example 1 of EP0789081) were used. Comparison of sensitivities corresponding to HHV-6 are shown in Figure 2 below. Figure 3 provides further amplification fragments obtained under above-described experimental conditions.

## Claims

1. A method for detection of HSV'-1, HSV-2 and VZV, comprising amplification in a single tube of a sample comprising viral genetic material with the pairs of primers:
HSV1S-18 (CCTTCGAACAGCTCCTGG) (SEQ ID NO: .3) and HSV1-AS (ATGACGCCGATGTACTTTTTCTT) (SEQ ID NO: 2);
HSV2S-20 (TCCATTTTCGTTTTGTGCCG) (SEQ ID NO; 4) and HSV1-AS (ATGACGCCGATGTACTTTTTCTT) (SEQ ID NO: 2); and
VV1S (AAGGTTATATATGGAGATACGGA) (SEQ ID NO: 9) and VV1AS (ATTACCCCAATGTACTTTTTCTT) (SEQ ID NO. 10),
to obtain amplification products comprising portions of HSV-1, HSV-2 and VZV genetic material, if present.

2. The method of claim 1 further comprising denaturing the amplification products and hybridizing the products with target-specific probes.

3. The method of claims 1 and 2 comprising:
a) contacting the sample with the set of amplification primers;
b) subjecting the test sample mixed with the set of amplification primers to a nucleic acid amplification reaction, the amplification reaction being intended to amplify target sequences present in the sample;
c) obtaining single-stranded oligonucleotides from any amplification products;
d) allowing single stranded oligonucleotides of c) to hybridise with a plurality of target-specific probes, and
e) detecting hybridised oligonucleotides.

4. The method of any preceding claim wherein the primer HSV1S-18 (SEQ ID NO: 3) is unlabelled and the primer HSV1-AS (SEQ ID NO: 2) is labelled, so that the relevant amplification product comprises one labelled amplification strand and one unlabelled amplification strand.

5. The method of claim 4 further comprising denaturation of the amplification products; and hybridisation of the amplification products with one or more probes which may be target-specific and non-target-specific probes; **characterised in that** the labelled strand obtained from the labelled primer HSV1-AS (SEQ ID NO: 2) can hybridise to a target-specific probe, and the unlabelled strand obtained from the unlabelled primer HSV1S-18. (SEQ ID NO: 3) can hybridise to the non-target-specific probe (if present).

6. A kit for the detection and identification in a test sample of HSV-1, HSV-2 and VZV, said kit comprising:
i) a nucleic acid amplification mix comprising the pairs of amplification primers HSV1S-18 (SEQ ID NO: 3) and HSV1-AS (SEQ ID NO: 2), HSV2S-20 (SEQ ID NO: 4) and HSV1-AS (SEQ ID NO: 2), and VV1S (SEQ ID NO: 9) and VV1AS (SEQ ID NO: 10)
ii) an array vessel or set of array vessels, each comprising a microarray wherein target-specific probes are provided,
iii) reagents for use in visualising hybridisation of nucleic acids to the probes of the microarray.

7. The kit according to claim 6, further comprising reagents for nucleic acid extraction and/or purification.

8. The kit according to claims 6 and 7 wherein the primer pair constituted by HSV1S-18 and HSV1-AS comprises primer HSV1S-18 (SEQ ID NO: 3) unlabelled, and primer HSV1-AS (SEQ ID NO: 2) labelled.

9. The kit of any of claim 6 to 8 further comprising, as internal control, amplification primers RTS (GCTTGGGCGTGTCTCAAAATCT) (SEQ ID NO: 20) and RTA (GTCGCCACGGTTGATGAGAGCT) (SEQ ID NO: 21), as well as a nucleic acid amplifiable by such primers.

10. The kit of any of claims 6 to 9 wherein the array vessel is an array tube.

11. The kit of any of claims 6 to 10 wherein the set of array vessels is an array strip.

12. The kit of any of claims 6 to 11 wherein the probe molecules of the microarray are printed on a solid support, the solid support being the bottom of an array vessel.

13. The kit of any of claims 6 to 12 wherein the probe molecules of the microarray are printed on a solid support, the solid support being attached to the bottom of an array vessel.

14. The kit of any of claims 6 to 13 wherein the microarray comprises probes that bind to their corresponding target sequences under the same hybridisation conditions.

15. Use of the method according to claims 1 to 5, or of the kit according to claims 6 to 14. for the detection and identification, if present in a test sample, of one or more viral agents selected from the group comprising HSV-1, HSV-2 and VZV.

## Patentansprüche

1. Verfahren zum Nachweis von HSV-1, HSV-2 und VZV, umfassend Amplifikation in einem einzigen Röhrchen von einer Probe, umfassend virales genetisches Material, mit den Paaren von Primern:
HSV1S-18 (CCTTCGAACAGCTCCTGG) (SEQ ID NO: 3) und HSV1-AS (ATGACGCCGATGTACTTTTTCTT) (SEQ ID NO: 2);
HSV2S-20 (TCCATTTTCGTTTTGTGCCG) (SEQ ID NO: 4) und HSV1-AS (ATGACGCCGATGTACTTTTTCTT) (SEQ ID NO: 2); und
VV1S (AAGGTTATATATGGAGATACGGA) (SEQ ID NO: 9) und VV1AS (ATTACCCCAATGTACTTTTTCTT) (SEQ ID NO: 10),
um Amplifikationsprodukte, umfassend Anteile des genetischen Materials von HSV-1, HSV-2 und VZV, wenn vorhanden, zu erhalten.

2. Verfahren nach Anspruch 1, weiterhin umfassend Denaturieren der Amplifikationsprodukte und Hybridisieren der Produkte mit Ziel-spezifischen Sonden.

3. Verfahren nach den Ansprüchen 1 und 2, umfassend:
a) Inkontaktbringen der Probe mit dem Satz von Amplifikationsprimern;
b) Unterwerfen der Testprobe, gemischt mit dem Satz von Amplifikationsprimern, einer Nucleinsäure-Amplifikationsreaktion, wobei die Amplifikationsreaktion Zielsequenzen, vorhanden in der Probe, amplifizieren soll;
c) Erhalten von einzelsträngigen Oligonucleotiden aus allen Amplifikationsprodukten;
d) Erlauben, dass einzelsträngige Oligonucleotide von c) mit einer Mehrzahl von Ziel-spezifischen Sonden hybridisieren, und
e) Nachweisen von hybridisierten Oligonucleotiden.

4. Verfahren nach einem vorhergehenden Anspruch, wobei der Primer HSV1S-18 (SEQ ID NO: 3) unmarkiert ist und der Primer HSV1-AS (SEQ ID NO: 2) markiert ist, so dass das relevante Amplifikationsprodukt einen markierten Amplifikationsstrang und einen unmarkierten Amplifikationsstrang umfasst.

5. Verfahren nach Anspruch 4, weiterhin umfassend Denaturierung der Amplifikationsprodukte; und Hybridisierung der Amplifikationsprodukte mit einer oder mehreren Sonden, welche Ziel-spezifische und nicht-Ziel-spezifische Sonden sein können; **dadurch gekennzeichnet, dass** der markierte Strang, erhalten von dem markierten Primer HSV1-AS (SEQ ID NO: 2) mit einer Ziel-spezifischen Sonde hybridisieren kann und der unmarkierte Strang, erhalten von dem unmarkierten Primer HSV1S-18 (SEQ ID NO: 3) mit der nicht-Ziel-spezifischen Sonde (wenn vorhanden) hybridisieren kann.

6. Kit für den Nachweis und die Identifikation in einer Testprobe von HSV-1, HSV-2 und VZV, wobei der Kit umfasst:
i) ein Nucleinsäure-Amplifikationsgemisch, umfassend die Paare von Amplifikationsprimern HSV1S-18 (SEQ ID NO: 3) und HSV1-AS (SEQ ID NO: 2), HSV2S-20 (SEQ ID NO: 4) und HSV1-AS (SEQ ID NO: 2) und VV1S (SEQ ID NO: 9) und VV1AS (SEQ ID NO: 10),
ii) ein Array-Gefäß oder einen Satz von Array-Gefäßen, jedes umfassend einen Microarray, wobei Ziel-spezifische Sonden bereitgestellt werden,
iii) Reagenzien zur Verwendung beim Visualisieren der Hybridisierung von Nucleinsäuren mit den Sonden des Microarrays.

7. Kit gemäß Anspruch 6, weiterhin umfassend Reagenzien für Nucleinsäureextraktion und/oder -reinigung.

8. Kit gemäß den Ansprüchen 6 und 7, wobei das Primerpaar, gebildet durch HSV1S-18 und HSV1-AS, den unmarkierten Primer HSV1S-18 (SEQ ID NO: 3) und den markierten Primer HSV1-AS (SEQ ID NO: 2) umfasst.

9. Kit nach einem der Ansprüche 6 bis 8, weiterhin umfassend, als interne Kontrolle, die Amplifikationsprimer RTS (GCTTGGGCGTGTCTCAAAATCT) (SEQ ID NO: 20) und RTA (GTCGCCACGGTTGATGAGAGCT) (SEQ ID NO: 21) ebenso wie eine Nucleinsäure, amplifizierbar durch derartige Primer.

10. Kit nach einem der Ansprüche 6 bis 9, wobei das Array-Gefäß ein Array-Röhrchen ist.

11. Kit nach einem der Ansprüche 6 bis 10, wobei der Satz von Array-Gefäßen ein Array-Streifen ist.

12. Kit nach einem der Ansprüche 6 bis 11, wobei die Sondenmoleküle des Microarrays auf einen festen Träger gedruckt sind, wobei der feste Träger der Boden eines Array-Gefäßes ist.

13. Kit nach einem der Ansprüche 6 bis 12, wobei die Sondenmoleküle des Microarrays auf einen festen Träger gedruckt sind, wobei der feste Träger an den Boden eines Array-Gefäßes angefügt ist.

14. Kit nach einem der Ansprüche 6 bis 13, wobei der Microarray Sonden umfasst, die unter den gleichen Hybridisierungsbedingungen an ihre entsprechenden Zielsequenzen binden.

15. Verwendung des Verfahrens gemäß den Ansprüchen 1 bis 5, oder des Kits gemäß den Ansprüchen 6 bis 14, für den Nachweis und die Identifikation von, wenn vorhanden in einer Testprobe, einem oder mehreren viralen Mitteln, ausgewählt aus der Gruppe, umfassend HSV-1, HSV-2 und VZV.

## Revendications

1. Procédé de détection du HSV-1, HSV-2 et VZV, comprenant l'amplification dans un seul tube d'un échantillon comprenant de la matière génétique virale avec les paires d'amorces:
HSV1S-18 (CCTTCGAACAGCTCCTGG) (SEQ ID NO: 3) et HSV1-AS (ATGACGCCGATGTACTT TTTCTT) (SEQ ID NO: 2);
HSV2S-20 (TCCATTTTCGTTTTGTGCCG) (SEQ ID NO; 4) et HSV1-AS (ATGACGCCGATGTACTT TTTCTT) (SEQ ID NO: 2); et
VV1S (AAGGTTATATATGGAGATACGGA) (SEQ ID NO: 9) et VV1AS (ATTACCCCAATGTACTTT TTCTT) (SEQ ID NO. 10).
afin d'obtenir des produits d'amplification comprenant des portions de matière génétique de HSV-1, HSV-2 et VZV, si présentes.

2. Procédé selon la revendication 1, comprenant en outre dénaturer les produits d'amplification et hybrider les produits avec des sondes cible-spécifiques.

3. Procédé selon les revendications 1 et 2, comprenant:
a) mettre l'échantillon en contact avec le jeu d'amorces d'amplification;
b) soumettre l'échantillon de test mélangé avec le jeu d'amorces d'amplification à une réaction d'amplification d'acide nucléiques, la réaction d'amplification étant destinée à amplifier les séquences cibles présentes dans l'échantillon;
c) obtenir des oligonucléotides simple brin à partir de produits d'amplification quelconques;
d) permettre aux oligonucléotides simple brin de c) s'hybrider avec une pluralité de sondes cible-spécifiques, et
e) détecter les oligonucléotides hybridés.

4. Procédé selon l'une quelconque des revendications précédentes, dans lequel l'amorce HSV1S-18 (SEQ ID NO: 3) est non marquée et l'amorce HSV1-AS (SEQ 10 NO: 2) est marquée, de sorte que le produit d'amplification pertinent comprend un brin d'amplification marqué et un brin d'amplification non marqué.

5. Procédé selon la revendication 4, comprenant en outre la dénaturation des produits d'amplification et l'hybridation des produits d'amplification avec une ou plusieurs sondes qui peuvent être des sondes cible-spécifiques et non cible-spécifiques; **caractérisé en ce que** le brin marqué obtenu à partir de l'amorce marquée HSV1-AS (SEQ ID NO: 2) peut s'hybrider à une sonde cible-spécifique et **en ce que** le brin non marqué obtenu à partir de l'amorce non marquée HSV1S-18 (SEQ ID NO: 3) peut s'hybrider à la sonde non-cible spécifique (si présente).

6. Kit de détection et d'identification de HSV-1, HSV-2 et VZV dans un échantillon de test, ledit kit comprenant:
i) un mélange d'amplification d'acides nucléiques comprenant les paires d'amorces d'amplification HSV1S-18 (SEQ ID NO: 3) et HSV1-AS (SEQ ID NO: 2), HSV2S-20 (SEQ ID NO: 4) et HSV1-AS (SEQ ID NO: 2), et VV1S (SEQ ID NO: 9) et VV1AS (SEQ ID NO: 10)
ii) un réacteur à biopuce ou une série de réacteurs à biopuce, comprenant chacun une biopuce dans laquelle les sondes cible-spécifiques sont fournies,
iii) des réactifs à utiliser dans la visualisation de l'hybridation des acides nucléiques aux sondes de la biopuce.

7. Kit selon la revendication 6, comprenant en outre des réactifs pour l'extraction et/ou la purification d'acides nucléiques.

8. Kit selon les revendications 6 et 7, dans lequel la paire d'amorces constituée par HSV1S-18 et HSV1-AS comprend l'amorce HSV1S-18 (SEQ ID NO: 3) non marquée et l'amorce HSV1-AS (SEQ ID NO: 2) marquée.

9. Kit selon l'une quelconque des revendications 6 à 8, comprenant en qualité de témoin interne, les amorces d'amplification RTS (GCTTGGGCGTGTCTCAAAATCT) (SEQ ID NO: 20) et RTA (GTCGCCACGGTTGATGAGAGCT) (SEQ ID NO: 21), ainsi qu'un acide nucléique amplifiable par de telles amorces.

10. Kit selon l'une quelconque des revendications 6 à 9, dans lequel le réacteur à biopuce est un tube à biopuce.

11. Kit selon l'une quelconque des revendications 6 à 10, dans lequel la série de réacteurs à biopuce est une bande de biopuces.

12. Kit selon l'une quelconque des revendications 6 à 11, dans lequel les molécules sondes de la biopuce sont imprimées sur un support solide, le support solide formant le fond d'un réacteur à biopuce.

13. Kit selon l'une quelconque des revendications 6 à 12, dans lequel les molécules sondes de la biopuce sont imprimées sur un support solide, le support solide étant attaché au fond d'un réacteur à biopuce.

14. Kit selon l'une quelconque des revendications 6 à 13, dans lequel la biopuce comprend des sondes qui se lient à leurs séquences cibles correspondantes dans les mêmes conditions d'hybridation.

15. Utilisation du procédé selon les revendications 1 à 5 ou du kit selon les revendications 6 à 14, pour détecter et identifier un ou plusieurs agents viraux sélectionnés parmi le groupe comprenant HSV-1, HSV-2 et VZV, si présents dans un échantillon de test.
